(19) Europäisches Patentamt / European Patent Office / Office européen des brevets

(11) **EP 2 744 899 B1**

(12) **FASCICULE DE BREVET EUROPEEN**

(45) Date de publication et mention
de la délivrance du brevet:
**24.02.2016  Bulletin 2016/08**

(21) Numéro de dépôt: **12758533.9**

(22) Date de dépôt: **02.08.2012**

(51) Int Cl.:
***C12N 9/42*** *(2006.01)*

(86) Numéro de dépôt international:
**PCT/FR2012/000328**

(87) Numéro de publication internationale:
**WO 2013/026964 (28.02.2013 Gazette 2013/09)**

(54) **PROCÉDÉ DE PRODUCTION DE CELLULASES PAR UN CHAMPIGNON FILAMENTEUX ADAPTÉ À UN FERMENTEUR AYANT UN FAIBLE COEFFICIENT DE TRANSFERT VOLUMETRIQUE D'OXYGÈNE KLA**

VERFAHREN ZUR HERSTELLUNG VON CELLULASEN DURCH EINEN AN EINEN FERMENTER ADAPTIERTEN FADENPILZ MIT NIEDRIGEM VOLUMETRISCHEN SAUERSTOFFÜBERTRAGUNGSKOEFFIZIENTEN KLA

METHOD FOR THE PRODUCTION OF CELLULASES BY A FILAMENTOUS FUNGUS ADAPTED TO A FERMENTER HAVING A LOW VOLUMETRIC OXYGEN TRANSFER COEFFICIENT KLA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **19.08.2011  FR 1102556**

(43) Date de publication de la demande:
**25.06.2014  Bulletin 2014/26**

(73) Titulaires:
  • **IFP Énergies nouvelles**
    **92852 Rueil-Malmaison Cedex (FR)**
  • **Institut National de la Recherche Agronomique**
    **75338 Paris (FR)**
  • **Agro Industrie Recherches et Developpements**
    **51110 Pomacle (FR)**

(72) Inventeurs:
  • **BEN CHAABANE, Fadhel**
    **75020 Paris (FR)**
  • **JOURDIER, Etienne**
    **75014 Paris (FR)**
  • **COHEN, Céline**
    **75017 Paris (FR)**
  • **CHAUSSEPIED, Bernard**
    **28130 Hanches (FR)**

(56) Documents cités:
  EP-A1- 1 690 944     WO-A1-2009/026716
  FR-A1- 2 555 603     FR-A1- 2 881 753

  • **POURQUIÉ J ET AL: "Cellulase production by Trichoderma reesei", 1 janvier 1993 (1993-01-01), BIOCONVERSION OF FOREST AND AGRICULTURAL PLANT RESIDUES, CAB INTERNATIONAL, GB, PAGE(S) 107 - 116, XP009141741, ISBN: 978-0-85198-798-9 le document en entier**

EP 2 744 899 B1

## Description

### DOMAINE DE L'INVENTION

L'invention est relative à un procédé de production de cellulases par un champignon filamenteux, nécessaire pour l'hydrolyse enzymatique de biomasse lignocellulosique mise en oeuvre par exemple dans les procédés de production de biocarburants dits de seconde génération ou d'autres procédés des industries chimiques, papetières ou textiles.

### ART ANTÉRIEUR

La mise au point de procédés économiquement viables de production de biocarburants de 2ème génération est aujourd'hui un vaste sujet d'actualité. Ces derniers sont produits à partir de biomasse lignocellulosique et posent moins de problèmes de concurrence d'usage des terres agricoles avec l'alimentaire, par rapport aux biocarburants dits de première génération qui sont produits à partir de canne à sucre, maïs, blé ou betterave.

La biomasse ligno-cellulosique se caractérise par une structure complexe constituée de trois principales fractions: la cellulose, les hémicelluloses et les lignines. De façon classique, le procédé de sa transformation en éthanol comprend plusieurs étapes. Le prétraitement permet de rendre la cellulose accessible aux enzymes qui sont des cellulases. L'étape d'hydrolyse enzymatique permet la transformation de la cellulose en glucose qui est ensuite transformée en éthanol lors de l'étape de fermentation par en général la levure *Saccharomyces cerevisiae.* Enfin l'étape de distillation va permettre de séparer et récupérer l'éthanol du moût de fermentation.

Les différentes études technico-économiques démontrent que la réduction du coût des cellulases est un des points-clés des procédés de production biologique d'éthanol à partir des matières premières lignocellulosiques. A l'heure actuelle, les cellulases industrielles sont principalement produites par un champignon filamenteux, *Trichoderma reesei,* en raison de son fort pouvoir de sécrétion. La stratégie qui est appliquée industriellement est de faire une croissance rapide du champignon jusqu'à une concentration donnée puis d'induire la production de cellulases afin de maximiser la productivité et le rendement. *Trichoderma reesei* est aérobie strict et sa croissance aboutit à une forte augmentation de la viscosité du milieu qui rend difficile le transfert d'oxygène nécessaire à sa survie. Ce dernier est lié au $K_La$ qui est appelé coefficient de transfert volumétrique d'oxygène ramené à l'unité de volume de milieu. C'est le produit du coefficient $K_L$ (coefficient d'échange global pour $O_2$ en $m.s^{-1}$ ou $m.h^{-1}$) et du coefficient "a" (aire d'échange spécifique ramenée à l'unité de volume de phase liquide de milieu de culture, en $m^2$ par $m^3$ de milieu de culture). Le $K_La$ est proportionnel à l'agitation et à l'aération. Afin de satisfaire la demande biologique en oxygène qui augmente durant la phase de croissance de champignon, il est nécessaire d'augmenter le transfert d'oxygène qui se fait généralement par une augmentation de l'agitation ou du débit d'aération. Ceci aboutit à une augmentation des dépenses énergétiques du procédé (puissance du moteur pour l'agitation et compresseur pour l'aération) avec, par conséquent, une augmentation des coûts opératoires. Les coûts liés à l'agitation et à l'aération peuvent représenter jusqu'à 50 % des coûts opératoires du procédé de production de cellulases.

Une des voies de diminution du coût de production des enzymes est donc d'abaisser les coûts opératoires en modifiant la conduite du procédé de façon à minimiser le $K_La$ nécessaire tout en maintenant la productivité en cellulases. Cela permet en outre de simplifier l'étude de mise à l'échelle (ou "scale-up" en anglais) pour le dimensionnement du fermenteur industriel (typiquement, de 100 à 1000 $m^3$) qui devient difficile pour des valeurs de $K_La$ supérieures à 200 $h^{-1}$.

Les enzymes du complexe enzymatique de *Trichoderma reesei* contiennent trois grands types d'activités : les endoglucanases, les exoglucanases et les cellobiases ou beta-glucosidases. D'autres protéines possédant des fonctions ou activités indispensables à l'hydrolyse des matériaux ligno-cellulosiques sont également produites par *Trichoderma reesei,* les xylanases par exemple. La présence d'un substrat inducteur est indispensable à l'expression des enzymes cellulolytiques et/ou hémicellulolytiques.

La régulation des gènes de cellulases sur différentes sources de carbone a été étudiée dans le détail. Ils sont induits en présence de cellulose, de ses produits d'hydrolyse (exemple: cellobiose) ou de certains oligosaccharides comme le lactose ou le sophorose (Ilmén et al., 1997; Appl.Environ. Microbiol. 63: 1298-1306). Les techniques de génétique classique par mutation ont permis la sélection de souches de *Trichoderma reesei* hyperproductrices de cellulases telles que les souches MCG77 (Gallo - brevet US 4275 167), MCG 80 (Allen, A.L. et Andreotti, R.E., Biotechnol- Bioengi 1982, 12, 451-459 1982), RUT C30 (Montenecourt, B.S. et Eveleigh, D.E., Appl. Environ. Microbiol. 1977, 34, 777-782) et CL847 (Durand et al, 1984, Proc. Colloque SFM "Génétique des microorganismes industriels". Paris. H. HESLOT Ed, pp 39-50).

Pour obtenir de bonnes productivités en enzymes, les procédés industriels de production de cellulases comme par exemple celui décrit dans le brevet FR 2 881 753 se font en deux étapes:

- une étape de croissance en mode "batch" où il est nécessaire d'apporter une source de carbone rapidement assimilable pour la croissance de *Trichoderma reesei,* puis

- une étape de production en mode "fed-batch" utilisant un substrat inducteur comme par exemple le lactose qui permet l'expression des cellulases et la sécrétion dans le milieu de culture. L'alimentation en continu de ces substrats carbonés solubles, en limitant la concentration résiduelle dans les cultures et en optimisant la quantité de sucre, permet d'obtenir de fortes productivités enzymatiques. Le flux optimal de source carbonée appliqué dans le brevet cité est compris entre 35 et 45 mg (de sucre).g (de poids sec cellulaire)$^{-1}$.h$^{-1}$.

Ce protocole présente l'inconvénient de nécessiter une forte dépense en énergie pour répondre à la demande en oxygène du micro-organisme. En fin de phase de croissance, la demande en oxygène est très élevée. En effet, lorsque tous les substrats sont disponibles en excès, la croissance se fait à un taux de croissance proche du taux de croissance maximal de la souche. La demande biologique en oxygène qui est fonction du taux de croissance et de la concentration en biomasse va augmenter. Comme les cultures se font en régulant la concentration en oxygène dissous à une valeur constante, la vitesse de transfert d'oxygène VTO doit être égale à la vitesse de consommation d'oxygène VCO (ou demande biologique en oxygène).

$$k_La*(O_2*-O_{2L}) = (\mu*X)/R_{X/O2}$$

avec:

O$_2$* : concentration en oxygène à saturation
O$_{2L}$ : concentration en oxygène dans la phase liquide
$\mu$: taux de croissance du microorganisme (h$^{-1}$)
R$_{X/O2}$ : rendement de biomasse cellulaire par rapport à l'oxygène consommé
X = concentration en biomasse (poids sec) cellulaire

Ce type de procédé nécessite donc un k$_L$a très élevé pour satisfaire à cette demande. Cela se fait en général par une augmentation de l'agitation (ou de l'aération) consommatrice d'énergie électrique.
Avec un k$_L$a 2 fois plus faible, la biomasse maximale qu'il est possible d'atteindre avec le même taux de croissance est 2 fois plus faible. Il est possible d'atteindre la même quantité de biomasse avec un taux de croissance plus faible mais cela nécessite plus de temps et donc entraîne une baisse la productivité finale en enzymes sécrétées.
La présente invention permet de mieux contrôler la demande biologique en oxygène sans diminuer la productivité en enzymes. Cela a été rendu possible par l'exploitation des caractéristiques physiologiques de champignons filamenteux tels que *Trichoderma reesei* en condition de limitation par le substrat carboné.

**RÉSUMÉ DE L'INVENTION**

La présente invention concerne un procédé de production de cellulases par un champignon filamenteux permettant de maintenir les performances en productivité en enzymes en utilisant un bioréacteur ayant un faible coefficient de transfert volumétrique d'oxygène k$_L$a.

**DESCRIPTION DES DESSINS**

La Figure 1 représente une comparaison des valeurs simulées par le modèle avec les valeurs expérimentales citées dans l'article de Tolan et Foody (1999) obtenues avec une autre souche de *Trichoderma reesei*

La Figure 2 représente l'évolution de la concentration en biomasse, en protéines et du k$_L$a correspondant à l'exemple 1.

La Figure 3 représente l'évolution des variables d'état au cours du temps correspondant à l'exemple 2.

La Figure 4 représente l'évolution des variables d'état au cours du temps correspondant à l'exemple 3.

La Figure 5 représente l'évolution du k$_L$a au cours du temps correspondant à l'exemple 3.

**DESCRIPTION DÉTAILLÉE DE L'INVENTION**

La présente invention porte sur un procédé de production de cellulases par une souche de champignon filamenteux, en bioréacteur agité et aéré comprenant au moins deux étapes :

- la première étape de croissance en présence d'au moins un substrat carboné de croissance en phase batch avec une concentration en substrat carboné de croissance comprise entre 10 et 80 g/L.

- une deuxième étape de croissance et de production en présence d'au moins un substrat carboné inducteur en phase fed-batch en présence d'un flux limitant de source de carbone compris entre 50 et 140 mg par gramme de cellules et par heure.

De façon préférée, la concentration en substrat carboné de croissance est comprise entre 10 et 20 g/L. Encore plus préférentiellement, elle est comprise entre 12 et 17 g/L.
Pendant la phase de croissance, la concentration en substrat carboné est telle que la croissance se fait à taux de croissance maximal.
De façon préférée, la concentration en substrat carboné inducteur utilisé en phase fed batch est comprise entre 70 et 100 mg par gramme de cellules et par heure. Encore plus préférentiellement, elle est comprise entre 80 et 90 mg par gramme de cellules et par heure.

Le bioréacteur utilisé dans la présente invention peut ainsi avoir un coefficient de transfert volumétrique d'oxygène $k_L a$ compris entre 40 et 180 $h^{-1}$, de préférence entre 40 et 150 $h^{-1}$.

La seconde étape est réalisée en condition de limitation du substrat carboné inducteur avec un flux inférieur à la capacité maximale de consommation de la souche.

Le substrat carboné de croissance est choisi de préférence parmi le lactose, le glucose, le xylose, les résidus obtenus après fermentation éthanolique des sucres monomères des hydrolysats enzymatiques de biomasse cellulosique, et/ou un extrait brut de pentoses hydrosolubles provenant du prétraitement d'une biomasse cellulosique.

Le substrat carboné inducteur est choisi de préférence parmi le lactose, le cellobiose, le sophorose, les résidus obtenus après fermentation éthanolique des sucres monomères des hydrolysats enzymatiques de biomasse cellulosique, et/ou un extrait brut de pentoses hydrosolubles provenant du prétraitement d'une biomasse cellulosique.

Les substrats de croissance et inducteurs cités ci-dessus peuvent être utilisés seuls ou en mélange. Selon sa nature, le substrat carboné de croissance choisi pour l'obtention de la biomasse est introduit dans le fermenteur avant stérilisation ou est stérilisé séparément et introduit dans le bioréacteur après stérilisation.

Le substrat carboné inducteur introduit pendant la phase fed batch est stérilisé de façon indépendante, avant d'être introduit dans le réacteur.

De façon préférée, quand la source de carbone inductrice est le lactose, la solution aqueuse est préparée à la concentration de 200-250 $g.L^{-1}$.

Le procédé selon la présente invention permet d'obtenir une productivité analogue en cellulases en utilisant un bioréacteur ayant une capacité de transfert d'oxygène deux fois et demi plus faible, soit un $K_L a$ de 100 $h^{-1}$ au lieu de 250 $h^{-1}$. La corrélation reliant le $K_L a$ à la puissance dissipée dans des bioréacteurs aérés et agités, telle que celle indiquée par exemple dans le rapport NREL "Lignocellulosic Biomass to Ethanol Process Design and economic utilizing co-current Dilute acide prehydrolysis and enzymatic hydrolysis current and futuristic scenarios", R Wooley et al. NREL/TP-580-26157 (1999), section production d'enzymes, est la suivante :

$$K_L a = 0{,}026*(P/V)^{0.4}*V_G^{0.5}$$

avec:

P/V : puissance dissipée en $W/m^3$
$V_G$ : vitesse superficielle de gaz (m/s).

Ainsi, selon cette corrélation, la puissance dissipée (PN) pour un $K_L a$ de 100 $h^{-1}$ est environ 10 fois inférieure à celle nécessaire lorsque le $K_L a$ vaut 250 $h^{-1}$.

L'avantage du procédé selon la présente invention est de permettre une simplification de l'extrapolation du procédé à l'échelle industrielle (typiquement de 100 à 1000 $m^3$) et une réduction des coûts opératoires.

Le procédé est simple, robuste et exploite les propriétés physiologiques du champignon en conditions de limitation par le substrat carboné.

Le mode de conduite a été adapté par rapport au procédé classique, d'une part en diminuant la concentration initiale en substrat de croissance lors de la première phase du procédé en mode « batch » afin de diminuer la demande biologique maximale en oxygène à la fin de cette phase et d'autre part en augmentant le flux de substrat carboné lors de la phase « fed-batch » pour continuer, durant le début de cette phase, à produire, à un taux de croissance réduit, de la biomasse cellulaire en même temps que les enzymes. Les propriétés physiologiques du champignon sont exploitées pour déterminer le débit de fed-batch et la concentration en biomasse désirée.

Cela permet de maintenir les performances finales en productivité du procédé tout en nécessitant des capacités de transfert d'oxygène moindres du bioréacteur.

La souche mise en oeuvre dans le procédé est une souche d'un champignon filamenteux appartenant aux genres *Trichoderma, Aspergillus, Penicillium ou Schizophyllum,*
De façon préférée, les souches utilisées sont des souches appartenant à l'espèce *Trichoderma reesei.*

Les souches industrielles utilisées appartiennent à l'espèce *Trichoderma reesei,* éventuellement modifiées pour améliorer les enzymes cellulolytiques et/ou hémicellulolytiques par les procédés de mutation-sélection, comme par exemple la souche IFP CL847 ; les souches améliorées par les techniques de recombinaison génétique peuvent être également utilisées. Ces souches sont cultivées en fermenteurs agités et aérés dans des conditions compatibles avec leur croissance et la production des enzymes. D'autres souches de microorganismes produisant des enzymes selon des processus similaires à ceux utilisés pour *Trichoderma* peuvent être utilisées.

De façon préférée, la souche utilisée est une souche de *Trichoderma reesei* modifiée par mutation, sélection ou recombinaison génétique.

Par exemple, la souche est une souche CL847, RutC30, MCG77, ou MCG80.

Un flux de substrat carboné inducteur qs supérieur à environ 140mg de sucre par g de biomasse par h entraîne une accumulation de sucre dans le milieu et modifie le comportement physiologique de *Trichoderma* résultant en une baisse de la vitesse spécifique de production de protéines qp (phénomène de répression catabolique). En limitant ce flux à des valeurs comprises entre 50 et 140 mg de sucre par gramme de biomasse et par heure, la souche produit simultanément, lorsqu'elle est en condition de limitation, de la biomasse et des enzymes, pour tendre vers un état d'équilibre où elle ne fait plus que des enzymes.

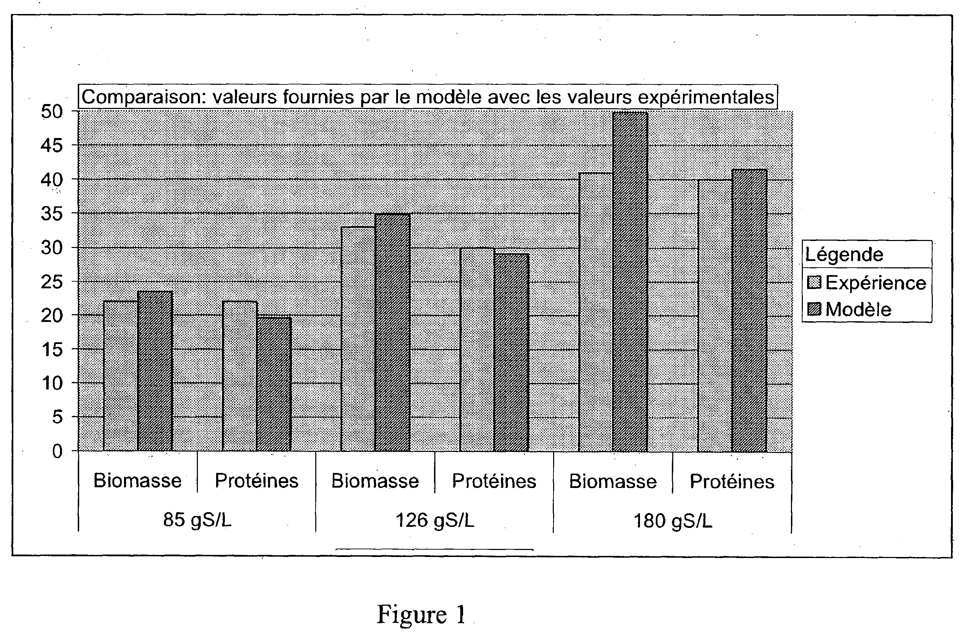

Un modèle a été établi sur la base de ces observations et a été appliqué pour simuler la production de biomasse et d'enzymes réalisée en continu à un taux de dilution de 0.018 h$^{-1}$ pour différentes concentrations initiales en substrat carboné. Les conditions correspondent à des travaux décrits dans un article de Tolan et Foody ("Cellulase from submerged fermentation", (1999), Adv. in biochemical engineering biotechnology, Vol 65, p42-67) qui citent les travaux de Nicholson et al. (1989) (Proceedings 7th Canadian bioenergy seminar, Energy Mines, and Resources) et fournissent les résultats expérimentaux des concentrations en biomasse et en protéines. Nous avons gardé les paramètres cinétiques identifiés de la souche CL847 même si les auteurs utilisent une souche différente. Les résultats sont reportés sur la figure 1 et démontre une très bonne adéquation entre le modèle et l'expérience.

Cela démontre également que ce modèle est valable pour d'autres souches de *Trichoderma reesei* qui ont le même comportement physiologique que la souche CL847.

La concentration en substrat carboné de croissance durant la phase batch a été baissée par rapport à l'enseignement de l'art antérieur (FR 2 881753) pour diminuer la demande biologique maximale en oxygène à la fin de cette phase (à $\mu$max) pour un bioréacteur ayant un $k_L$a de 100 h$^{-1}$. Le flux de substrat carboné est ensuite augmenté lors de la phase « fed-batch » par rapport au brevet FR 2 881 753 qui recommandait un flux compris entre 35 et 45 mg de substrat carboné inducteur par gramme de biomasse et par heure. Cela permet de continuer à produire de la biomasse en même temps que les enzymes durant le début de cette phase mais à un taux de croissance réduit, ce qui permet de contrôler la demande biologique en oxygène. Le flux de source de carbone pendant la phase fed-batch est donc augmenté à une valeur supérieure à 50 mg de sucre par gramme de biomasse et par heure en début de phase de fed-batch. La croissance se poursuit en même temps que la production en enzymes et se stabilise lorsque le flux de source carbonée sera proche de l'optimal de la souche.

## EXEMPLES

Parmi les exemples qui suivent, l'exemple 1 présente une culture utilisant les conditions de référence du brevet FR 2 881 753 avec un bioréacteur ayant un $k_L$a de 250 h$^{-1}$ L'exemple 2 présente une expérience réalisée dans les mêmes conditions que l'exemple 1 avec un fermenteur ayant un $k_L$a de 100 h$^{-1}$. Cet exemple aboutit à une accumulation de substrat carboné avec une forte production de biomasse et une faible production d'enzymes. L'exemple 3 est celui mettant en oeuvre le procédé selon la présente invention. Il permet d'obtenir une productivité analogue à celle de l'exemple 1 avec un bioréacteur ayant un $k_L$a de 100 h$^{-1}$.

Exemple 1 : Production d'enzymes sur glucose

La production de cellulases est effectuée en fermenteur agité mécaniquement. Le milieu minéral a la composition suivante : KOH 1,66 g./L, H3PO4 85 % 2 mL/L, $(NH_4)_2SO_4$ 2,8 g/L, $MgSO_4$, 7 $H_2O$ 0,6 g/L, $CaCL_2$ 0,6 g/L, $MnSO_4$ 3,2 mg/L, $ZnSO_4$, 7 $H_2O$ 2,8 mg/L, $CoCl_2$ 10 4,0 mg/L, $FeSO_4$, 7 $H_2O$ 10 mg/L, Corn Steep 1,2 g/L, anti-mousse 0,5 mL/L.

Le fermenteur contenant le milieu minéral est stérilisé à 120 °C pendant 20 minutes, la source carbonée est une solution de glucose stérilisée à part à 120°C pendant 20 minutes puis ajoutée stérilement dans le fermenteur de façon à avoir une concentration finale de 30 g/L. Le fermenteur est ensemencé à 10% (v/v) avec une préculture liquide de la souche de *Trichoderma reesei* CL847. Le milieu minéral de la préculture, est identique à celui du fermenteur mise à part l'addition de phtalate de potassium à 5 g/L pour tamponner le pH. La croissance du champignon en préculture est faite en utilisant le glucose comme substrat carboné, à la concentration de 30 g.L$^{-1}$. La croissance de l'inoculum dure de 2 à 3 jours et est effectuée à 28 °C dans un incubateur agité à pression atmosphérique. Le transfert vers le fermenteur est réalisé si la concentration résiduelle en glucose est inférieure à 15 g/L

L'expérience effectuée en bioréacteur comporte deux phases :

- Une phase de croissance sur substrat carboné glucose (concentration initiale = 30 g/L) à une température de 27 °C et un pH de 4,8 (régulé par une solution d'ammoniaque 5,5 M). L'aération est de 0,5 vvm et l'agitation est augmentée entre 200 et 800 rpm en fonction de la $pO_2$ (pression en oxygène dissous), qui est régulée à 30%.

- Une phase de production d'enzymes. Après 30 heures, la solution de lactose à 250 g.L$^{-1}$ est injectée en continu au débit de 35 mg par g de cellules et par heure jusqu'à 250 heures. La température est baissée à 25 °C et le pH à 4 jusqu'à la fin de la culture. Le pH est régulé par addition d'une solution d'ammoniaque à 5,5 N qui apporte l'azote nécessaire à la synthèse des protéines excrétées. La teneur en oxygène dissous est maintenue au-dessus de 30 % par action de l'agitation

La production d'enzymes est suivie par le dosage des protéines extracellulaires par la méthode de Lowry basée sur un étalonnage réalisé avec du BSA ("Bovine serum albumine"), après séparation du mycélium par filtration ou centrifugation. Les activités cellulolytiques déterminées sont:

- L'activité papier filtre ou FP, exprimée en UPF (unité papier filtre) qui permet de doser l'activité globale du pool enzymatique endoglucanases et exoglucanases

- Les activités β-glucosidase et xylanases pour les activités spécifiques.

L'activité FP est mesurée sur papier Whatman n° 1 (Procédure recommandée par la commission biotechnologique IUPAC) à la concentration initiale de 50 g.$L^{-1}$ ; on détermine la prise d'essai de la solution enzymatique à analyser qui libère l'équivalent de 2 g.$L^{-1}$ de glucose (dosage colorimétrique) en 60 minutes. Le principe de l'activité papier filtre est de déterminer par dosage au DNS (acide dinitrosalicylique) la quantité de sucres réducteurs issue d'un papier Whatman N°1.

Le substrat utilisé pour déterminer l'activité β-glucosidase est le p-nitrophenyl-ß-D-glucopyranoside (PNPG). Il est clivé par la ß-glucosidase qui libère le p-nitrophenol. Une unité d'activité β-glucosidase est définie comme la quantité d'enzyme nécessaire pour produire 1μmol de p-nitrophenol à partir de PNPG par minute et est exprimé en IU/mL.

Le principe du dosage de l'activité xylanase repose sur la détermination par dosage DNS de la quantité de sucres réduits issue de la solution de xylane hydrolysée. Cette méthode de dosage utilise les propriétés réductrices des sucres et principalement du xylose. L'activité xylanase est exprimée en IU/mL et correspond à la quantité d'enzyme nécessaire pour produire 1μmol de xylose par minute.

Les activités spécifiques sont obtenues en divisant les activités exprimées en IU/mL par la concentration en protéines. Elles sont exprimées en IU.$mg^{-1}$.

Les $K_L a$ sont déterminés à partir des bilans gazeux après vérification des bilans carbone et redox.

Les déterminations analytiques sur le moût final de l'exemple 1 donnent les résultats suivants :

Biomasse cellulaire g/L 15,5
Protéines g/L 50,1
Productivité = 0,20 g/L/h
UPF 29,1 IU/mL
Xylanase spécifique 8,2 IU/mg
β-Glucosidase spécifique 1,0 IU/mg

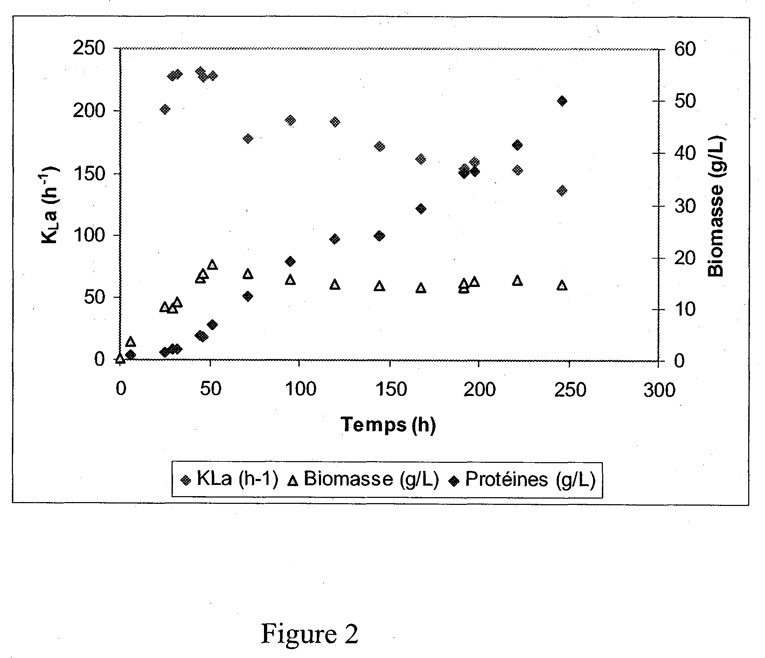

La figure 2 reprend l'évolution de la concentration en biomasse, en protéines et du $K_L a$ au cours du temps. On constate que la biomasse cellulaire augmente jusqu'à 15 g/L durant les 50 premières heures de l'expérience et que le $K_L a$ est de 240 $h^{-1}$. La concentration en protéines augmente légèrement durant les 50 premières heures puis fortement à partir du moment où la concentration en biomasse est stable. Elle atteint 50 g/L à la fin de la culture.

Exemple 2:

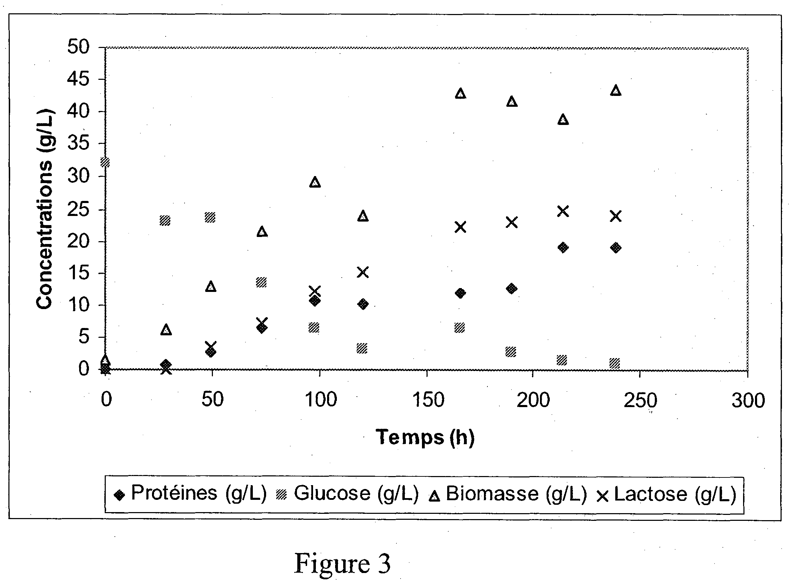

L'exemple 2 est réalisé dans les mêmes conditions que l'exemple 1 sauf que le fermenteur utilisé a un $K_L a$ maximal de 100 $h^{-1}$ et que son volume initial est de 750 mL. La fermentation aboutit à une forte production de biomasse cellulaire (45 g/L) et à une faible production de protéines (19 g/L) (voir Figure 3).

Cela est dû à la présence de concentrations résiduelles importantes de substrat carboné (lactose, glucose) tout au long de l'expérience. Celui-ci réprime la production de cellulases qui est induite en condition de limitation de substrat carboné inducteur. Le glucose n'est pas consommé car les capacités de transfert d'oxygène du bioréacteur sont 2,5 fois plus faibles, ce qui diminue la vitesse de consommation de substrat carboné qui est proportionnelle à la vitesse de consommation d'$O_2$.

En effet la vitesse de transfert d'oxygène est exprimée de la manière suivante:

$$VTO = K_L a\ (O_2{}^* - O_{2L})$$

avec:

$O_2{}^*$ : concentration en oxygène à saturation
$O_{2L}$ : concentration en oxygène dans la phase liquide

La vitesse de consommation d'oxygène est donc limitée par la VTO qui est 2,5 fois plus faible

Les déterminations analytiques sur le moût final donnent les résultats suivants :

Biomasse cellulaire g/L 45
Protéines g/L 19
UPF 10,1 IU/mL
Xylanase spécifique 8,5 IU/mg
β-Glucosidase spécifique 1,2 IU/mg

Exemple 3 (selon l'invention):

Le bioréacteur ayant un $k_L a$ de 100 $h^{-1}$ est utilisé mais le procédé de production est modifié pour être mis en oeuvre selon la présente invention.

La concentration en glucose initiale est ainsi abaissée à 15 g/L de façon à ce que la vitesse biologique maximale de consommation de dioxygène soit compatible avec le fermenteur utilisé. Le rendement de production de biomasse cellulaire par rapport au glucose est de 0,5 g/g lorsque celui-ci est présent en excès. Cela veut dire que la vitesse maximale de consommation de dioxygène pour cette quantité de glucose est de 0,5 g.$L^{-1}$.$h^{-1}$ (pour un taux de croissance maximale de 0,08 $h^{-1}$ et un rendement de conversion de dioxygène en biomasse de 1,2 g/g.

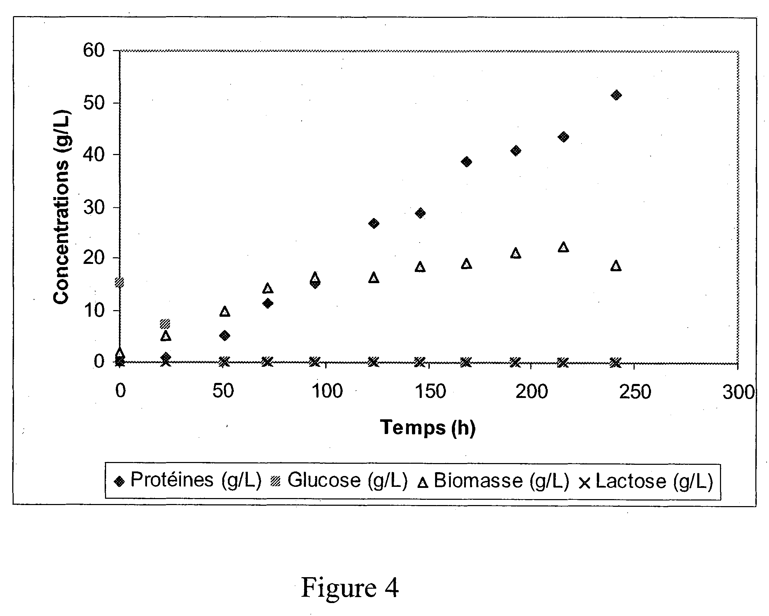

La phase fed-batch est lancée après 24 heures avec un flux de 89 mg de substrat par gramme de biomasse et par heure (solution de lactose à 250 g/L). La phase de croissance se poursuit en même temps que la production de protéines. Cette dernière atteint 51,7 g/L après 240 heures d'expérience (Figure 4). La productivité finale en protéines est donc maintenue malgré l'utilisation de fermenteur ayant des capacités de transfert réduites. Elle est de 0,21 g/L/h (elle était de 0,20 g/L/h dans le cas de l'exemple 1).

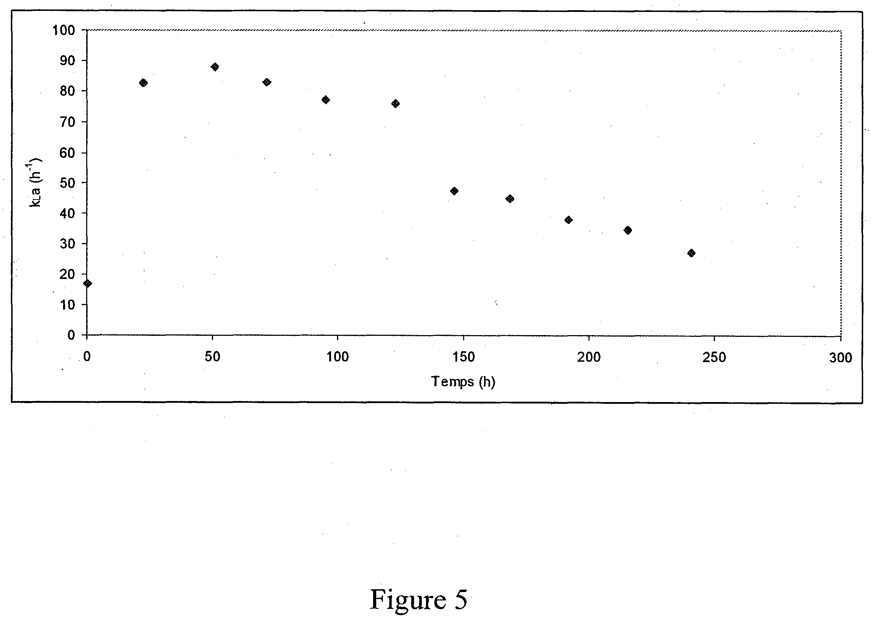

La figure 5 illustre l'évolution du $k_L a$ au cours de l'expérience qui reste inférieur à 100 h$^{-1}$.

Les déterminations analytiques sur le moût final donnent les résultats suivants :

Biomasse cellulaire g/L 18,9
Protéines g/L 51,7
Productivité = 0,21 g/L/h
UPF 30,1 IU/mL
Xylanase spécifique 9,5 IU/mg
β-GLucosidase spécifique 1,12 IU/mg

**Revendications**

1. Procédé de production de cellulases par une souche appartenant à un champignon filamenteux, en bioréacteur agité et aéré comprenant au moins deux étapes :

   - la première étape de croissance en présence d'au moins un substrat carboné de croissance en phase batch étant réalisée avec une concentration en substrat carboné de croissance comprise entre 10 et 60 g/L
   - une deuxième étape de croissance et de production d'enzymes en présence d'au moins un substrat carboné inducteur alimenté en phase fed-batch en présence d'un flux limitant de source de carbone compris entre 50 et 140 mg par gramme de biomasse cellulaire et par heure,
   - et le bioréacteur a un coefficient de transfert volumétrique d'oxygène $k_L a$ compris entre 40 et 180 h$^{-1}$.

2. Procédé selon la revendication 1 dans lequel la concentration en substrat carboné de croissance est comprise entre 10 et 20 g/L.

3. Procédé selon la revendication 2 dans lequel la concentration en substrat carboné est comprise entre 12 et 17 g/L.

4. Procédé selon l'une des revendications précédentes dans lequel le flux de source de carbone est compris entre 70 et 100 mg par gramme de biomasse cellulaire et par heure.

5. Procédé selon la revendication 4 dans lequel le flux de source de carbone est compris entre 80 et 90 mg par gramme de biomasse et par heure.

6. Procédé selon l'une des revendications précédentes dans lequel le bioréacteur a un coefficient de transfert volumétrique d'oxygène $k_L a$ compris entre 40 et 150 h$^{-1}$

7. Procédé selon l'une des revendications précédentes dans lequel le substrat carboné de croissance est choisi parmi le lactose, le glucose, le xylose, les résidus obtenus après fermentation éthanolique des sucres monomères des hydrolysats enzymatiques de biomasse cellulosique, et/ou un extrait brut de pentoses hydrosolubles provenant du prétraitement d'une biomasse cellulosique

8. Procédé selon l'une des revendications précédentes dans lequel le substrat carboné inducteur est choisi parmi le lactose, le cellobiose, le sophorose, les résidus obtenus après fermentation éthanolique des sucres monomères des hydrolysats enzymatiques de biomasse cellulosique, et/ou un extrait brut de pentoses hydrosolubles provenant du prétraitement d'une biomasse cellulosique.

9. Procédé selon l'une des revendications précédentes dans lequel le substrat carboné de croissance choisi pour l'obtention de la biomasse est introduit dans le fermenteur avant stérilisation.

10. Procédé selon l'une des revendications 1 à 9 dans lequel le substrat carboné de croissance choisi pour l'obtention de la biomasse est stérilisé séparément et introduit dans le bioréacteur après stérilisation.

11. Procédé selon l'une des revendications précédentes dans lequel le substrat carboné inducteur introduit pendant la phase fed-batch est stérilisé de façon indépendante, avant d'être introduite dans le réacteur.

12. Procédé selon l'une des revendications précédentes dans lequel la souche utilisée est une souche de *Trichoderma reesei.*

13. Procédé selon la revendication précédente dans lequel la souche utilisée est une souche de *Trichoderma reesei* modifiée par mutation sélection ou recombinaison génétique.

**Patentansprüche**

1. Verfahren zur Herstellung von Cellulasen durch eine zu einem Fadenpilz gehörenden Stamm in einem bewegten und belüfteten Bioreaktor umfassend mindestens zwei Stufen:

   - die erste Wachstumsstufe in Gegenwart we-

nigstens eines kohlenstoffhalten Wachstums-substrates in einer Batchphase wird durchgeführt mit einer Konzentration des kohlenstoffhaltigen Wachstumssubstrats zwischen 10 und 60 g/L,

- eine zweite Wachstums- und Enzymproduktionsstufe in Gegenwart wenigstens eines kohlenstoffhaltigen Induktionssubstrates in fed-BatchPhase in Gegenwart eines begrenzenden Flusses der Kohlenstoffquelle zwischen 50 und 140 mg pro Gramm zellularer Biomasse und pro Stunde,

- und der Bioreaktor hat einen volumetrischen Sauerstoffübertragungskoeffizienten $k_L a$ zwischen 40 und 180h$^{-1}$.

2. Verfahren nach Anspruch 1, bei dem die Konzentration des kohlenstoffhaltigen Wachstumssubstrates zwischen 10 und 20 g/L beträgt.

3. Verfahren nach Anspruch 2, bei dem die Konzentration des kohlenstoffhalten Substrates zwischen 12 und 17 g/L beträgt.

4. Verfahren nach einem der vorherigen Ansprüche, bei dem der Fluss der Kohlenstoffquelle zwischen 70 und 100 mg pro Gramm zellularer Biomasse und pro Stunde beträgt.

5. Verfahren nach Anspruch 4, bei dem der Fluss der Kohlenstoffquelle zwischen 80 und 90 mg pro Gramm zellularer Biomasse und pro Stunde beträgt.

6. Verfahren gemäß einem der vorherigen Ansprüche, bei dem der Bioreaktor einen volumetrischen Sauerstoffübertragungskoeffizienten $k_L a$ zwischen 40 und 150h$^{-1}$ aufweist.

7. Verfahren gemäß einem der vorherigen Ansprüche, bei dem das kohlenstoffhaltige Wachstumssubstrat ausgewählt ist aus Laktose, Glucose, Xylose, den nach ethanolischer Fermentation von monomeren Zuckern der enzymatischen Hydrolysate der zellulosehaltigen Biomasse erhaltenen Rückstände und/oder dem Rohextrakt der aus der Vorbehandlung einer zellulosehaltigen Biomasse stammenden wasserlöslichen Pentosen.

8. Verfahren gemäß einem der vorherigen Ansprüche, bei dem das kohlenstoffhaltige Induktionssubstrat ausgewählt ist aus Laktose, Zellobiose, Sophorose, den nach der ethanolischen Fermentation der monomeren Zucker der enzymatischen Hydrolysate von zellulosehaltiger Biomasse erhaltenen Rückstände, und/oder einem Rohextrakt der aus der Vorbehandlung einer zellulosehaltigen Biomasse stammenden wasserlöslichen Pentosen.

9. Verfahren gemäß einem der vorherigen Ansprüche, bei dem das zum Wachsen der Biomasse ausgewählte kohlenstoffhaltige Wachstumssubstrat in den Fermenter vor der Sterilisation eingeführt wird.

10. Verfahren nach einem der Ansprüche 1 bis 9, bei dem das zum Wachsen der Biomasse ausgewählte kohlenstoffhaltige Wachstumssubstrat getrennt sterilisiert wird und in den Bioreaktor nach Sterilisation eingeführt wird.

11. Verfahren gemäß einem der vorherigen Ansprüche, bei dem das während der fed-Batch Phase eingeführte kohlenstoffhaltige Induktionssubstrat vor dem Einführen in den Reaktor in unabhängiger Weise sterilisiert wird.

12. Verfahren gemäß einem der vorherigen Ansprüche, bei dem der verwendete Stamm *Trichoderma reesei* ist.

13. Verfahren gemäß einem der vorherigen Ansprüche, bei dem der verwendete Stamm ein durch genetische Mutation, Selektion oder Rekombination modifizierter Stamm von *Trichoderma reesei* ist.

## Claims

1. A process for the production of cellulases using a strain belonging to a filamentous fungus in a stirred, aerated bioreactor, comprising at least two steps:

   • the first growth step in the presence of at least one carbonaceous growth substrate in batch phase, carried out with a concentration of carbonaceous growth substrate in the range 10 to 60 g/L;
   • a second step for growth and enzyme production in the presence of at least one inducer carbonaceous substrate in fed batch phase in the presence of a limiting flow of carbon source in the range 50 to 140 mg per gram of cellular biomass per hour,
   • and the bioreactor has a volumetric oxygen transfer coefficient, $k_L a$, in the range 40 to 180 h$^{-1}$.

2. A process according to claim 1, in which the concentration of carbonaceous growth substrate is in the range 10 to 20 g/L.

3. A process according to claim 2, in which the concentration of carbonaceous substrate is in the range 12 to 17 g/L.

4. A process according to one of the preceding claims, in which the flow of the carbon source is in the range 70 to 100 mg per gram of cellular biomass per hour.

5. A process according to claim 4, in which the flow of the carbon source is in the range 80 to 90 mg per gram of cellular biomass per hour.

6. A process according to one of the preceding claims, in which the bioreactor has a volumetric oxygen transfer coefficient, $k_La$, in the range 40 to 150 h$^{-1}$.

7. A process according to one of the preceding claims, in which the carbonaceous growth substrate is selected from lactose, glucose, xylose, residues obtained after ethanolic fermentation of monomeric sugars of enzymatic hydrolysates of cellulosic biomass and/or a crude extract of hydrosoluble pentoses deriving from pre-treatment of a cellulosic biomass.

8. A process according to one of the preceding claims, in which the inducer carbonaceous substrate is lactose, cellobiose, sophorose, residues obtained after ethanolic fermentation of monomeric sugars of enzymatic hydrolysates of cellulosic biomass and/or a crude extract of hydrosoluble pentoses deriving from pre-treatment of a cellulosic biomass.

9. A process according to one of the preceding claims, in which the carbonaceous growth substrate selected for producing biomass is introduced into the fermenter before sterilization.

10. A process according to one of claims 1 to 9, in which the carbonaceous growth substrate selected for producing the biomass is sterilized separately and introduced into the bioreactor after sterilization.

11. A process according to one of the preceding claims, in which the inducer carbonaceous substrate introduced during the fed batch phase is sterilized independently before being introduced into the reactor.

12. A process according to one of the preceding claims, in which the strain used is a strain of *Trichoderma reesei.*

13. A process according to one of the preceding claims, in which the strain used is a strain of *Trichoderma reesei* modified by genetic mutation, selection or recombination.

Comparaison: valeurs fournies par le modèle avec les valeurs expérimentales

Légende
- Expérience
- Modèle

Biomasse | Protéines | Biomasse | Protéines | Biomasse | Protéines
85 gS/L | 126 gS/L | 180 gS/L

Figure 1

Figure 2

Figure 3

Figure 4

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4275167 A, Gallo

- FR 2881753

**Littérature non-brevet citée dans la description**

- **ILMÉN et al.** *Appl.Environ. Microbiol.,* 1997, vol. 63, 1298-1306
- **ALLEN, A.L. ; ANDREOTTI, R.E.** *Biotechnol- Bioengi 1982,* 1982, vol. 12, 451-459
- **MONTENECOURT, B.S. ; EVELEIGH, D.E.** *Appl. Environ. Microbiol.,* 1977, vol. 34, 777-782
- **DURAND et al.** Proc. Colloque SFM. 1984, 39-50

- **R WOOLEY et al.** Lignocellulosic Biomass to Ethanol Process Design and economic utilizing co-current Dilute acide prehydrolysis and enzymatic hydrolysis current and futuristic scenarios. *NREL/TP-580-26157,* 1999
- **TOLAN ; FOODY.** Cellulase from submerged fermentation. *Adv. in biochemical engineering biotechnology,* 1999, vol. 65, 42-67
- **NICHOLSON et al.** *Proceedings 7th Canadian bioenergy seminar, Energy Mines, and Resources,* 1989